# DEMANDE DE BREVET EUROPEEN

(11) **EP 1 690 522 A1**
(43) Date de publication de la demande: **16.08.2006**
(21) Numéro de dépôt: 06100132.7
(22) Date de dépôt: 06.01.2006
(51) Int. Cl.: A61K 8/06, A61K 8/43, A61Q 19/00, A61K 9/107, A61K 47/16

(54) **Nanoémulsion cosmétique contenant un composé d'urée hydroxylé**

(30) Priorité: 11.02.2005 FR 0501413
(71) Demandeur: L'ORÉAL, 75008 Paris (FR)
(72) Inventeur: Simonnet, Jean-Thierry, 94240, Cachan (FR)
(74) Mandataire: Kromer, Christophe

(57) **Abrégé**

L'invention concerne une nanoémulsion huile-dans-eau comportant une phase huileuse dispersée dans une phase aqueuse, dont les globules d'huile ont une taille moyenne en nombre inférieure à 100 nm, comprenant :
- (i) au moins un lipide amphiphile comprenant au moins un lipide amphiphile non ionique et éventuellement un lipide amphiphile ionique, la phase huileuse et le lipide amhiphile étant présents en une teneur telle que le rapport pondéral phase huileuse/lipide amphiphile va de 3 à 10,
- et (ii) au moins un dérivé d'urée hydroxylé.

Application en cosmétique, notamment soin et au maquillage des matières kératiniques, en dermatologie, en cosmétique, en ophtalmologie.

## Description

La présente invention concerne une nanoémulsion, comprenant au moins un lipide amphiphile non ionique, et éventuellement un lipide amphiphile anionique, et au moins un dérivé d'urée, ainsi qu'à l'utilisation de ladite nanoémulsion en application topique notamment dans les domaines cosmétique et dermatologique, et dans les domaines pharmaceutique et/ou ophtalmologique.

Les émulsions huile-dans-eau (H/E) sont bien connues dans le domaine de la cosmétique et de la dermatologie, notamment pour la préparation de produits cosmétiques tels que des laits, des crèmes, des toniques, des sérums, des eaux de toilette.

Les nanoémulsions sont des émulsions H/E caractérisées par une taille des globules huileux inférieure à 100 nm, les globules huileux étant stabilisés par une couronne de lipides amphiphiles pouvant éventuellement former une phase cristal liquide de type lamellaire, situés à l'interface huile/phase aqueuse. La transparence de ces émulsions provient de la petite taille des globules huileux, petite taille obtenue grâce à l'utilisation d'une énergie mécanique et notamment d'un homogénéisateur haute pression. Les nanoémulsions sont à différencier des microémulsions de par leur structure. En effet, les microémulsions sont des dispersions thermodynamiquement stables constituées de micelles de lipide(s) amphiphile(s) gonflées par de l'huile. De plus, les microémulsions ne nécessitent pas d'énergie mécanique importante pour être réalisées ; elles se forment spontanément par simple mise en contact des constituants. Les inconvénients majeurs des microémulsions sont liés à leur forte proportion en tensioactifs, conduisant à des intolérances et entraînant un toucher collant lors de l'application sur la peau. Par ailleurs, leur domaine de formulation est en général très étroit et leur stabilité en température très limitée.

Les nanoémulsions comprennent un ou plusieurs lipide(s) amphiphile(s). Par lipide amphiphile, on entend ici toutes molécules ayant une structure bipolaire, c'est-à-dire comportant au moins une partie hydrophobe et au moins une partie hydrophile et ayant la propriété de réduire la tension superficielle de l'eau (γ< 55mN/m) et de réduire la tension interfaciale entre l'eau et une phase huileuse. Les synonymes de lipide amphiphile sont par exemple : tensioactif, agent de surface, émulsionnant.

Les documents EP-A-728 460 et EP-A-780 114 décrivent des nanoémulsions à base de lipides amphiphiles non ioniques liquides ou de tensioactifs siliconés. Des nanoémulsions sont également décrites dans les documents FR-A-2,787,026, FR-A-2,787,027, FR-A-2,787,325, FR-A-2,787,326, FR-A-2,787,703, FR-A-2,787,728.

Pour favoriser l'obtention de gouttelettes d'huile ayant une taille inférieure à 100 nm, on utilise de façon connue des solvants tels que l'éthanol ou les glycols comme le propylène glycol, le butylène glycol ou le dipropylène glycol. Or ces solvants ont l'inconvénient de conférer de mauvaises propriétés cosmétiques à la nanoémulsion, essentiellement en raison du caractère collant de la nanoémulsion, perçu en particulier lors de l'application sur la peau. De plus, ces nanoémulsions ne présentent pas de propriété hydratante de la peau satisfaisante.

Le but de la présente invention est donc de disposer de nanoémulsion stables, notamment pendant un mois à 45 °C, ne présentant pas d'effet collant notamment lors de leur application sur les matières kératiniques, et en particulier sur la peau. Un autre but de l'invention est de disposer d'une nanoémulsion ayant une bonne propriété hydratante pour la peau.

Les inventeurs ont découvert qu'une telle composition est obtenue en utilisant un composé d'urée particulier dans la nanoémulsion. Le composé d'urée permet de réaliser un nanoémulsion stable, notamment pendant un mois à 45 °C. La nanoémulsion obtenue ne présente pas d'effet collant et permet d'hydrater la peau.

De façon plus précise, l'invention a pour objet une nanoémulsion huile-dans-eau comportant une phase huileuse dispersée dans une phase aqueuse, dont les globules d'huile ont une taille moyenne en nombre inférieure à 100 nm, caractérisée en ce qu'elle comprend :
- (i) au moins un lipide amphiphile comprenant au moins un lipide amphiphile non ionique et éventuellement un lipide amphiphile ionique, la phase huileuse et le lipide amhiphile étant présents en une teneur telle que le rapport pondéral phase huileuse/lipide amphiphile va de 3 à 10,
- et (ii) au moins un composé d'urée particulier.

Les nanoémulsions selon l'invention ont généralement un aspect transparent à bleuté. Leur transparence se mesure par un coefficient de transmittance à 600 nm allant de 10 à 90 % ou bien par une turbidité. La turbidité des compositions de l'invention va de 60 à 400 NTU et de préférence de 70 à 300 NTU, turbidité mesurée au turbidimètre portatif HACH - Modèle 2100 P à environ 25°C.

Les globules d'huile des nanoémulsions de l'invention ont une taille moyenne en nombre, inférieure à 100 nm et de préférence allant de 20 à 80 nm et plus préférentiellement de 40 à 60 nm. La diminution de la taille des globules permet de favoriser la pénétration des actifs dans les couches superficielles de la peau (effet véhicule).

Les nanoémulsions conformes à l'invention sont préparées de préférence à des températures allant de 4 à 45°C et sont ainsi compatibles avec des actifs thermosensibles.

Le composé d'urée présent dans la composition selon l'invention est un composé de formule (I) suivante : 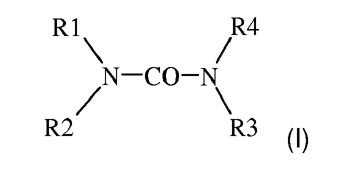 dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères.

Pour les composés de formule (I) :
- De préférence R₁ désigne un groupe hydroxyalkyle en C₂-C₆ et R₂, R₃, R₄ désignent, indépendamment l'un de l'autre, un atome d'hydrogène ou un groupe alkyle en C₁-C₄ ;
- Préférentiellement R₁ désigne un groupe hydroxyalkyle en C₂-C₆ comprenant de 1 à 5 groupes hydroxyles, notamment 1 groupe hydroxyle, et R₂, R₃, R₄ désignent un atome d'hydrogène ;
- Plus préférentiellement, R₁ désigne un groupe hydroxyalkyle en C₂-C₄ comprenant 1 groupe hydroxyle et R₂, R₃, R₄ désignent un atome d'hydrogène.

Parmi les groupes alkyle, on peut citer les groupes méthyle, éthyle, n-propyle, isopropyle, n-butyle, iso-butyle et tert-butyle.

Parmi les groupes hydroxyalkyle, on préfère ceux contenant un seul groupe hydroxyle et en particulier les groupes hydroxyéthyle, hydroxypropyle, hydroxybutyle, hydroxypentyle et hydroxyhexyle.

Parmi les sels, on peut citer les sels d'acides minéraux, tels que l'acide sulfurique, l'acide chlorhydrique, l'acide bromhydrique, l'acide iodhydrique, l'acide phosphorique, l'acide borique. On peut également citer les sels d'acides organiques, qui peuvent comporter un ou plusieurs groupes acide carboxylique, sulfonique, ou phosphonique. Il peut s'agir d'acides aliphatiques linéaires, ramifiés ou cycliques ou encore d'acides aromatiques. Ces acides peuvent comporter, en outre, un ou plusieurs hétéroatomes choisis parmi O et N, par exemple sous la forme de groupes hydroxyle. On peut notamment citer l'acide propionique, l'acide acétique, l'acide téréphtalique, l'acide citrique et l'acide tartrique.

Par solvat, on entend un mélange stoechiométrique dudit composé de formule (I) avec une ou plusieurs molécules d'eau ou de solvant organique, un tel mélange étant issu de la synthèse du composé de formule (I).

Comme composés de formule (1) préférés, on peut citer le N-(2-hydroxyéthyl)-urée ; le N-(2-hydroxypropyl)-urée ; le N-(3-hydroxypropyl)-urée ; le N-(2,3-dihydroxypropyl)- urée ; le N-(2,3,4,5,6-pentahydroxyhexyl)- urée ; le N-méthyl-N-(1,3,4,5,6-pentahydroxy-2-hexyl)- urée ; le N-méthyl-N'-(1-hydroxy-2-méthyl-2-propyl)- urée ; le N-(1-hydroxy-2-méthyl-2-propyl)- urée ; le N-(1,3-dihydroxy-2-propyl)- urée ; le N-(tris-hydroxyméthyl-méthyl)-urée ; le N-éthyl-N'-(2-hydroxyéthyl)- urée ; le N,N-bis-(2-hydroxyéthyl)- urée ; le N,N'-bis-(2-hydroxyéthyl)- urée ; le N,N-bis-(2-hydroxypropyl)- urée ; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)- urée ; le N-tert.Butyl-N'-(2-(hydroxyéthyl)-N'-(2-(hydroxypropyl)- urée ; le N-(1,3-dihydroxy-2-propyl)-N'-(2-hydroxyéthyl)- urée ; le N,N-Bis-(2-hydroxyéthyl)-N',N'-dimethyl- urée ; le N,N,N',N'-tetrakis-(2-hydroxyéthyl)- urée ; le N',N'-Bis-(2-hydroxyéthyl)-N',N'-bis-(2-hydroxypropyl)-urée ; et leurs mélanges.

De préférence, le composé de formule (I) est le N-(2-hydroxyéthyl)-urée.

Les composés de formule (I) sont des composés connus et notamment décrits dans la demande DE-A-2703185. Parmi ceux-ci, le N-(2-hydroxyéthyl)-urée est en outre disponible dans le commerce, sous forme de mélange à 50% en poids dans l'eau, auprès de la société NATIONAL STARCH sous la dénomination commerciale Hydrovance® .

Le composé de formule (I) peut être présent dans la nanoémulsion selon l'invention en une teneur allant de 1 % à 50 % en poids, par rapport au poids total de la composition, de préférence allant de 2 % à 25 % en poids, et préférentiellement allant de 2 % à 20 % en poids.

Les nanoémulsions selon l'invention comprennent au moins un lipide amphiphile non ionique.

Les lipides amphiphiles non ioniques de l'invention sont préférentiellement choisis parmi :
1/ les tensioactifs siliconés,
2/ les lipides amphiphiles liquides à température inférieure ou égale à 45°C, choisis parmi les esters d'au moins un polyol d'au moins un acide gras comportant au moins une chaîne alkyle en C₈-C₂₂, saturée ou non saturée, linéaire ou ramifiée, et notamment insaturée ou ramifiée, le polyol étant choisi dans le groupe formé par le polyéthylèneglycol comportant de 1 à 60 unités d'oxyde d'éthylène, le sorbitan, le glycérol pouvant comporter de 2 à 30 unités d'oxyde d'éthylène, les polyglycérols comportant de 2 à 15 unités de glycérol,
3/ les esters d'acide gras et de sucre et les éthers d'alcool gras et de sucre,
4/ les tensioactifs solides à une température égale à 45°C, choisis parmi les esters gras de glycérol, les esters gras de sorbitan et les esters gras de sorbitan oxyéthylénés, les éthers gras éthoxylés et les esters gras éthoxylés,
5/ Les copolymères blocs d'oxyde d'éthylène (A) et d'oxyde de propylène (B),
et les mélanges de ces tensioactifs.
1/ Les tensioactifs siliconés utilisables selon l'invention sont des composés siliconés comportant au moins une chaîne oxyéthylénée -OCH₂CH₂- et/ou oxypropylénée -OCH₂CH₂CH₂-. Comme tensioactifs siliconés pouvant être utilisés selon la présente invention, on peut citer ceux décrits dans les documents US-A-5,364,633 et US-A-5,411,744.
   De préférence, le tensioactif siliconé utilisé selon la présente invention est un composé de formule (II) : 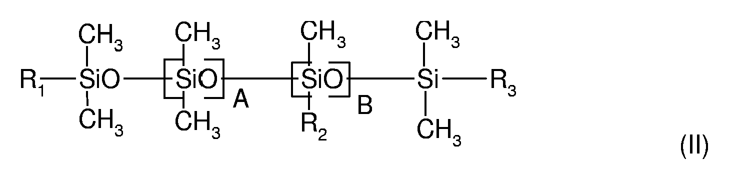 dans laquelle :
   R₁, R₂, R₃, indépendamment les uns des autres, représentent un radical alkyle en C₁-C₆ ou un radical -(CH₂)ₓ-(OCH₂CH₂)_{y}-(OCH₂CH₂CH₂)_{z}-OR₄, au moins un radical R₁, R₂ ou R₃ n'étant pas un radical alkyle ; R₄ étant un hydrogène, un radical alkyle ou un radical acyle ;
   A est un nombre entier allant de 0 à 200 ;
   B est un nombre entier allant de 0 à 50 ; à la condition que A et B ne soient pas égaux à zéro en même temps ;
   x est un nombre entier allant de 1 à 6 ;
   y est un nombre entier allant de 1 à 30 ;
   z est un nombre entier allant de 0 à 5.
   Selon un mode de réalisation préféré de l'invention, dans le composé de formule (X), le radical alkyle est un radical méthyle, x est un nombre entier allant de 2 à 6 et y est un nombre entier allant de 4 à 30.
   On peut citer, à titre d'exemple de tensioactifs siliconés de formule (II), les composés de formule (III) : 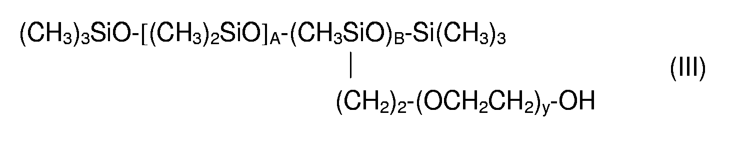 dans laquelle A est un nombre entier allant de 20 à 105, B est un nombre entier allant de 2 à 10 et y est un nombre entier allant de 10 à 20.
   On peut également citer à titre d'exemple de tensioactifs siliconés de formule (II), les composés de formule (IV) :

   H-(OCH₂CH₂)_{y}-(CH₂)₃-[(CH₃)₂SiO]_{A'}-(CH₂)₃-(OCH₂CH₂)_{y}-OH (IV)

   dans laquelle A' et y sont des nombres entiers allant de 10 à 20.
   On peut utiliser notamment comme tensioactifs siliconés, ceux commercialisés par la société Dow Corning sous les dénominations DC 5329, DC 7439-146, DC 2-5695 et Q4-3667. Les composés DC 5329, DC 7439-146, DC 2-5695 sont des composés de formule (XI) où respectivement A est 22, B est 2 et y est 12 ; A est 103, B est 10 et y est 12 ; A est 27, B est 3 et y est 12.
   Le composé Q4-3667 est un composé de formule (IV) où A est 15 et y est 13.
2/ Les lipides amphiphiles liquides à température inférieure ou égale à 45°C peuvent être notamment choisis parmi :
   - l'isostéarate de polyéthylèneglycol de poids molaire 400 (nom CTFA : PEG-8 Isostearate), vendu sous la dénomination Prisorine 3644 par la société UNICHEMA ;
   - l'isostéarate de diglycéryle, vendu par la société SOLVAY ;
   - le laurate de polyglycérol comportant 2 unités de glycérol (polyglyceryl-2 laurate), vendu sous la dénomination Diglycerin-monolaurate par la société SOLVAY ;
   - l'oléate de sorbitan, vendu sous la dénomination SPAN 80 par la société ICI ;
   - l'isostéarate de sorbitan, vendu sous la dénomination NIKKOL SI 10R par la société NIKKO ;
   - le cocoate d'α-butylglucoside ou le caprate d'α-butylglucoside commercialisés par la société ULICE.
3/ Les esters d'acide gras et de sucre, utilisables comme lipides amphiphiles non ioniques dans la nanoémulsion selon l'invention sont de préférence solides à une température inférieure ou égale à 45°C et peuvent être choisis notamment dans le groupe comprenant les esters ou les mélanges d'esters d'acide gras en C₈-C₂₂ et de sucrose, de maltose, de glucose ou de fructose, et les esters ou les mélanges d'esters d'acide gras en C₁₄-C₂₂ et de méthylglucose.
   Les acides gras en C₈-C₂₂ ou en C₁₄-C₂₂ formant le motif gras des esters utilisables dans la nanoémulsion de l'invention comportent une chaîne alkyle linéaire saturée ou non saturée, ayant respectivement de 8 à 22 ou de 14 à 22 atomes de carbone. Le motif gras des esters peut être notamment choisi parmi les stéarates, béhénates, arachidonates, palmitates, myristates, laurates, caprates et leurs mélanges. On utilise de préférence des stéarates.
   On peut citer, à titre d'exemple d'esters ou de mélanges d'esters d'acide gras et de sucrose, de maltose, de glucose ou de fructose, le monostéarate de sucrose, le distéarate de sucrose, le tristéarate de sucrose et leurs mélanges, tels que les produits commercialisés par la société Croda sous la dénomination Crodesta F50, F70, F110, F160 ayant respectivement un HLB (Hydrophilic Lipophilic Balance) de 5, 7, 11 et 16 ; et à titre d'exemple d'esters ou de mélanges d'esters d'acide gras et de méthylglucose, le distéarate de méthyl glucose et de polyglycérol-3, vendu par la société Goldschmidt sous la dénomination de Tego-care 450. On peut citer aussi les monoesters de glucose ou de maltose tels que l'o-hexadécanoyle-6-D-glucoside de méthyle et l'o-hexadécanoyle-6-D-maltoside.
   Les éthers d'alcool gras et de sucre, utilisables comme lipides amphiphiles non ioniques dans la nanoémulsion selon l'invention sont solides à une température inférieure ou égale à 45°C et peuvent être choisis notamment dans le groupe comprenant les éthers ou mélanges d'éthers d'alcool gras en C₈-C₂₂ et de glucose, de maltose, de sucrose ou de fructose et les éthers ou mélanges d'éthers d'alcool gras en C₁₄-C₂₂ et de méthylglucose. Ce sont notamment des alkylpolyglucosides.
   Les alcools gras en C₈-C₂₂ ou en C₁₄-C₂₂ formant le motif gras des éthers utilisables dans la nanoémulsion de l'invention comportent une chaîne alkyle linéaire saturée ou non saturée, ayant respectivement de 8 à 22 ou de 14 à 22 atomes de carbone. Le motif gras des éthers peut être notamment choisi parmi les motifs décyle, cétyle, béhényle, arachidyle, stéaryle, palmityle, myristyle, lauryle, capryle, hexadécanoyle, et leurs mélanges tels que cétéaryle.
   A titre d'exemple d'éthers d'alcool gras et de sucre, on peut citer les alkylpolyglucosides tels que le décylglucoside et le laurylglucoside commercialisés par exemple par la société Henkel sous les dénominations respectives de Plantaren 2000 et Plantaren 1200, le cétostéarylglucoside éventuellement en mélange avec l'alcool cétostéarylique, commercialisé par exemple sous la dénomination Montanov 68 par la société Seppic, sous la dénomination Tego-care CG90 par la société Goldschmidt et sous la dénomination Emulgade KE3302 par la société Henkel, ainsi que l'arachidylglucoside, par exemple sous la forme du mélange d'alcools arachidique et béhénique et d'arachidylglucoside, commercialisé sous la dénomination Montanov 202 par la société Seppic.
   On utilise plus particulièrement comme lipide amphiphile non ionique de ce type, le monostéarate de sucrose, le distéarate de sucrose, le tristéarate de sucrose et leurs mélanges, le distéarate de méthyl glucose et de polyglycérol-3 et les alkylpolyglucosides.
4/ Les esters gras de glycérol, utilisables comme lipides amphiphiles non ioniques dans la nanoémulsion selon l'invention, solides à une température égale à 45°C, peuvent être choisis notamment dans le groupe comprenant les esters formés d'au moins un acide comportant une chaîne alkyle linéaire saturée, ayant de 16 à 22 atomes de carbone, et de 1 à 10 motifs glycérol. On peut utiliser un ou plusieurs de ces esters gras de glycérol dans la nanoémulsion de l'invention.
   Ces esters peuvent être notamment choisis parmi les stéarates, béhénates, arachidates, palmitates et leurs mélanges. On utilise de préférence des stéarates et palmitates.
   On peut citer à titre d'exemple de tensioactif utilisable dans la nanoémulsion de l'invention, les monostéarate, distéarate, tristéarate et pentastéarate de décaglycérol (10 unités de glycérol) (noms CTFA : Polyglyceryl-10 stearate, Polyglyceryl-10 distearate, Polyglyceryl-10 tristearate, Polyglyceryl-10 pentastearate) tels que les produits vendus sous les dénominations respectives Nikkol Decaglyn 1-S, 2-S, 3-S et 5-S par la société Nikko, et le monostéarate de diglycérol (nom CTFA : Polyglyceryl-2 stearate) tel que le produit vendu par la société Nikko sous la dénomination Nikkol DGMS.
   Les esters gras de sorbitan, utilisables comme lipides amphiphiles non ioniques dans la nanoémulsion selon l'invention, solides à une température inférieure ou égale à 45°C, sont choisis notamment dans le groupe comprenant les esters d'acide gras en C₁₆-C₂₂ et de sorbitan et les esters d'acide gras en C₁₆-C₂₂ et de sorbitan oxyéthylénés. Ils sont formés d'au moins un acide gras comportant au moins une chaîne alkyle linéaire saturée, ayant respectivement de 16 à 22 atomes de carbone, et de sorbitol ou de sorbitol éthoxylé. Les esters oxyéthylénés comportent généralement de 1 à 100 unités d'oxyde d'éthylène et de préférence de 2 à 40 unités d'oxyde d'éthylène (OE).
   Ces esters peuvent être notamment choisis parmi les stéarates, béhénates, arachidates, palmitates, et leurs mélanges. On utilise de préférence des stéarates et palmitates.
   On peut citer à titre d'exemple d'ester gras de sorbitan et d'ester gras de sorbitan oxyéthyléné, utilisable dans la nanoémulsion de l'invention, le monostéarate de sorbitan (nom CTFA : Sorbitan stearate) vendu par la société ICI sous la dénomination Span 60, le monopalmitate de sorbitan (nom CTFA : Sorbitan palmitate) vendu par la société ICI sous la dénomination Span 40, le tristéarate de sorbitan 20 OE (nom CTFA : Polysorbate 65) vendu par la société ICI sous la dénomination Tween 65.
   Les éthers gras éthoxylés solides à une température inférieure ou égale à 45°C, utilisables comme lipides amphiphiles non ioniques dans la nanoémulsion selon l'invention sont de préférence des éthers formés de 1 à 100 unités d'oxyde d'éthylène et d'au moins une chaîne d'alcool gras ayant de 16 à 22 atomes de carbone. La chaîne grasse des éthers peut être notamment choisie parmi les motifs béhényle, arachidyle, stéaryle, cétyle, et leurs mélanges tels que cétéaryle. A titre d'exemple d'éthers gras éthoxylés, on peut citer les éthers d'alcool béhénique comprenant 5, 10, 20 et 30 unités d'oxyde d'éthylène (noms CTFA : Beheneth-5, Beheneth-10, Beheneth-20, Beheneth-30), tels que les produits commercialisés sous les dénominations Nikkol BB5, BB10, BB20, BB30 par la société Nikko, et l'éther d'alcool stéarylique comprenant 2 unités d'oxyde d'éthylène (nom CTFA : Steareth-2), tel que le produit commercialisé sous la dénomination Brij 72 par la société ICI.
   Les esters gras éthoxylés solides à une température inférieure ou égale à 45°C, utilisables comme lipides amphiphiles non ioniques dans la nanoémulsion selon l'invention sont des esters formés de 1 à 100 unités d'oxyde d'éthylène et d'au moins une chaîne d'acide gras comportant de 16 à 22 atomes de carbone. La chaîne grasse des esters peut être notamment choisie parmi les motifs stéarate, béhénate, arachidate, palmitate, et leurs mélanges. A titre d'exemple d'esters gras éthoxylés, on peut citer l'ester d'acide stéarique comprenant 40 unités d'oxyde d'éthylène, tel que le produit commercialisé sous la dénomination Myrj 52 (nom CTFA : PEG-40 stearate) par la société ICI ainsi que l'ester d'acide béhénique comprenant 8 unités d'oxyde d'éthylène (nom CTFA : PEG-8 behenate), tel que le produit commercialisé sous la dénomination Compritol HD5 ATO par la société Gattefosse.
5/ Les copolymères blocs d'oxyde d'éthylène et d'oxyde de propylène, utilisables comme lipides amphiphiles non ioniques dans la nanoémulsion selon l'invention peuvent être choisis notamment parmi les copolymères blocs de formule (V) :

   HO(C₂H₄O)x(C₃H₆O)y(C₂H₄O)zH (V)

   dans laquelle x, y et z sont des nombres entiers tels que x+z va de 2 à 100 et y va de 14 à 60, et leurs mélanges, et plus particulièrement parmi les copolymères blocs de formule (V) ayant un HLB allant de 2 à 16.

Ces copolymères blocs peuvent être notamment choisis parmi les poloxamers et notamment parmi le Poloxamer 231 tel que le produit commercialisé par la société ICI sous la dénomination Pluronic L81 de formule (V) avec x=z=6, y=39 (HLB 2) ; le Poloxamer 282 tel que le produit commercialisé par la société ICI sous la dénomination Pluronic L92 de formule (V) avec x=z=10, y=47 (HLB 6) ; et le Poloxamer 124 tel que le produit commercialisé par la société ICI sous la dénomination Pluronic L44 de formule (V) avec x=z=11, y=21 (HLB 16).

Comme lipides amphiphiles non ioniques, on peut aussi citer les mélanges de tensioactifs non ioniques décrits dans le document EP-A-705593 incorporé ici pour référence.

Parmi les lipides amphiphiles non ioniques, on peut utiliser en particulier :
- l'isostéarate de PEG 400 ou PEG-8 Isostéarate (comportant 8 moles d'oxyde d'éthylène),
- l'isostéarate de diglycéryle,
- le monolaurate de polyglycérol comportant 2 unités de glycérol et les stéarates de polyglycérol comportant 10 unités de glycérol,
- l'oléate de sorbitan,
- l'isostéarate de sorbitan,
et leurs mélanges.

Les lipides amphiphiles non ionique peuvent être présents dans la nanoémulsion selon l'invention en une teneur allant de 0,2 % à 12 % en poids, par rapport au poids total de la composition, et de préférence allant de 0,2 % à 8 % en poids, et préférentiellement allant de 0,2 % à 6 % en poids.

Selon une forme particulière de réalisation de l'invention, la nanoémulsion de l'invention peut contenir en outre un ou plusieurs lipides amphiphiles ioniques, en particulier un ou plusieurs lipides anioniques ou cationiques, différents des lipides amphiphiles non ioniques décrits précédemment. Leur ajout peut améliorer encore la stabilité de la dispersion.

Ainsi, les lipides amphiphiles anioniques pouvant être utilisés dans les nanoémulsions de l'invention sont choisis de préférence parmi :
- les sels alcalins du dicétyl- et du dimyristylphosphate ;
- les sels alcalins du cholestérol sulfate ;
- les sels alcalins du cholestérol phosphate ;
- les lipoaminoacides et leurs sels tels que les acylglutamates mono- et disodiques comme le sel disodique de l'acide N-stéaroyl L-glutamique commercialisé sous la dénomination Acylglutamate HS21 par la société AJINOMOTO ;
- les sels de sodium de l'acide phosphatidique ;
- les phospholipides ;
- les dérivés alkylsulfoniques notamment de formule (VI) : 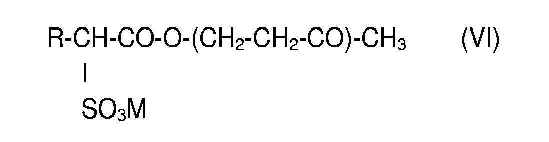
dans laquelle R représente des radicaux alkyle en C₁₆-C₂₂, en particulier les radicaux C₁₆H₃₃ et C₁₈H₃₇ pris en mélange ou séparément et M est un métal alcalin ou alcalino-terreux tel que le sodium ; et leurs mélanges.

Les lipides amphiphiles cationiques pouvant être utilisés dans les nanoémulsions de l'invention sont choisis, de préférence, dans le groupe formé par les sels d'ammonium quaternaire, les amines grasses et leurs sels.

Les sels d'ammonium quaternaires sont par exemple :
- ceux qui présentent la formule générale (VII) suivante : 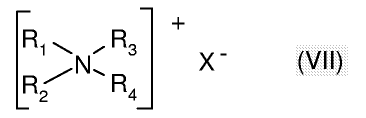
dans laquelle les radicaux R₁ à R₄, qui peuvent être identiques ou différents, représentent un radical aliphatique, linéaire ou ramifié, comportant de 1 à 30 atomes de carbone, ou un radical aromatique tel que aryle ou alkylaryle. Les radicaux aliphatiques peuvent comporter des hétéroatomes tels que notamment l'oxygène, l'azote, le soufre, les halogènes. Les radicaux aliphatiques sont par exemple choisis parmi les radicaux alkyle, alcoxy, polyoxyalkylène(C₂-C₆), alkylamide, alkyl(C₁₂-C₂₂)amido alkyle(C₂-C₆), alkyl(C₁₂-C₂₂)acétate, hydroxyalkyle, comportant environ de 1 à 30 atomes de carbone; X est un anion choisi dans le groupe des halogénures, phosphates, acétates, lactates, alkyl(C₂-C₆)sulfates, alkyl-ou-alkylarylsulfonates,

Parmi les sels d'ammonium quaternaire de formule (VIII), on préfère, d'une part, les chlorures de tétraalkylammonium comme par exemple les chlorures de dialkyldiméthylammonium ou d'alkyltriméthylammonium, dans lesquels le radical alkyl comporte environ de 12 à 22 atomes de carbone, en particulier les chlorures de béhényltriméthylammonium, de distéaryldiméthylammonium, de cétyltriméthylammonium, de benzyl diméthyl stéaryl ammonium ou encore, d'autre part, le chlorure de stéaramidopropyldiméthyl (myristyl acétate) ammonium commercialisé sous la dénomination «**CERAPHYL 70**» par la société **VAN DYK**. Le chlorure de béhényltriméthylammonium est le sel d'ammonium quaternaire le plus particulièrement préféré.
- les sels d'ammonium quaternaire de l'imidazolinium, comme par exemple celui de formule (VIII) suivante : 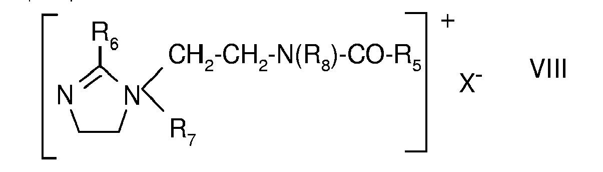
dans laquelle R₅ représente un radical alcényle ou alkyle comportant de 8 à 30 atomes de carbone par exemple dérivés des acides gras du suif, R₆ représente un atome d'hydrogène, un radical alkyle en C₁-C₄ ou un radical alcényle ou alkyle comportant de 8 à 30 atomes de carbone, R₇ représente un radical alkyle en C₁₋C₄ , R₈ représente un atome d'hydrogène, un radical alkyle en C₁-C₄, X est un anion choisi dans le groupe des halogénures, phosphates, acétates, lactates, alkylsulfates, alkyl-ou-alkylarylsulfonates. De préférence, R₅ et R₆ désignent un mélange de radicaux alcényle ou alkyle comportant de 12 à 21 atomes de carbone par exemple dérivés des acides gras du suif, R₇ désigne méthyle, R₈ désigne hydrogène. Un tel produit est par exemple commercialisé sous la dénomination «REWOQUAT W 75» par la société REWO,
- les sels de diammonium quaternaire de formule (IX) : 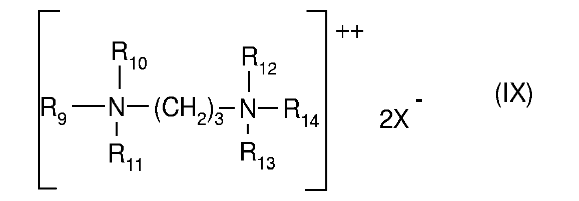
dans laquelle R₉ désigne un radical aliphatique comportant environ de 16 à 30 atomes de carbone, R₁₀, R₁₁, R₁₂, R₁₃ et R₁₄ , identiques ou différents sont choisis parmi l'hydrogène ou un radical alkyle comportant de 1 à 4 atomes de carbone, et X est un anion choisi dans le groupe des halogénures, acétates, phosphates, nitrates et méthylsulfates. De tels sels de diammonium quaternaire comprennent notamment le dichlorure de propanesuif diammonium.
- les sels d'ammonium quaternaire contenant au moins une fonction ester

Les sels d'ammonium quaternaire contenant au moins une fonction ester utilisables selon l'invention sont par exemple ceux de formule (X) suivante : 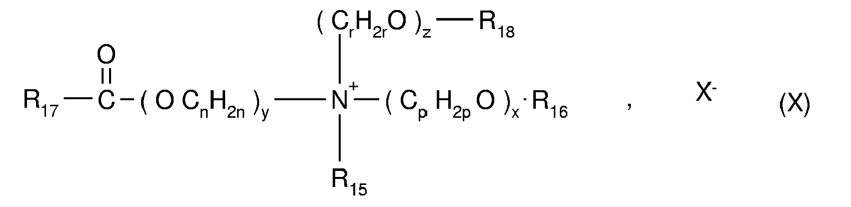 dans laquelle :
- R₁₅ est choisi parmi les radicaux alkyles en C₁-C₆ et les radicaux hydroxyalkyles ou dihydroxyalkyles en C₁-C₆ ;
- R₁₆ est choisi parmi :
   - le radical 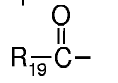
   - les radicaux R₂₀ hydrocarbonés en C₁-C₂₂ linéaires ou ramifiés, saturés ou insaturés,
   - l'atome d'hydrogène,
- R₁₈ est choisi parmi :
   - le radical 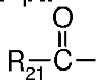
   - les radicaux R₂₂ hydrocarbonés en C₁-C₆ linéaires ou ramifiés, saturés ou insaturés,
   - l'atome d'hydrogène,
- R₁₇, R₁₉ et R₂₁, identiques ou différents, sont choisis parmi les radicaux hydrocarbonés en C₇-C₂₁, linéaires ou ramifiés, saturés ou insaturés ;
- n, p et r, identiques ou différents, sont des entiers valant de 2 à 6 ;
- y est un entier valant de 1 à 10 ;
- x et z, identiques ou différents, sont des entiers valant de 0 à 10 ;
- X- est un anion simple ou complexe, organique ou inorganique ;
sous réserve que la somme x + y + z vaut de 1 à 15 , que lorsque x vaut 0 alors R₁₆ désigne R₂₀ et que lorsque z vaut 0 alors R₁₈ désigne R₂₂.

Les radicaux alkyles R₁₅ peuvent être linéaires ou ramifiés et plus particulièrement linéaires.

De préférence R₁₅ désigne un radical méthyle, éthyle, hydroxyéthyle ou dihydroxypropyle et plus particulièrement un radical méthyle ou éthyle.

Avantageusement, la somme x + y + z vaut de 1 à 10.

Lorsque R₁₆ est un radical R₂₀ hydrocarboné, il peut être long et avoir de 12 à 22 atomes de carbone ou court et avoir de 1 à 3 atomes de carbone.

Lorsque R₁₈ est un radical R₂₂ hydrocarboné, il a de préférence 1 à 3 atomes de carbone.

Avantageusement, R₁₇, R₁₉ et R₂₁, identiques ou différents, sont choisis parmi les radicaux hydrocarbonés en C₁₁-C₂₁, linéaires ou ramifiés, saturés ou insaturés, et plus particulièrement parmi les radicaux alkyle et alcényle en C₁₁₋C₂₁, linéaires ou ramifiés, saturés ou insaturés.
De préférence, x et z, identiques ou différents, valent 0 ou 1.
Avantageusement, y est égal à 1.
De préférence, n, p et r, identiques ou différents, valent 2 ou 3 et encore plus particulièrement sont égaux à 2.

Dans la formule (X), l'anion X- est de préférence un halogénure (chlorure, bromure ou iodure) ou un alkylsulfate plus particulièrement méthylsulfate. On peut cependant utiliser le méthanesulfonate, le phosphate, le nitrate, le tosylate, un anion dérivé d'acide organique tel que l'acétate ou le lactate ou tout autre anion compatible avec l'ammonium à fonction ester. L'anion X- est encore plus particulièrement le chlorure ou le méthylsulfate.

On utilise plus particulièrement les sels d'ammonium de formule (X) dans laquelle :
- R₁₅ désigne un radical méthyle ou éthyle,
- x et y sont égaux à 1 ;
- z est égal à 0 ou 1 ;
- n, p et r sont égaux à 2 ;
- R₁₆ est choisi parmi :
   - le radical 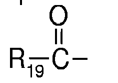
   - les radicaux méthyle, éthyle ou hydrocarbonés en C₁₄-C₂₂
   - l'atome d'hydrogène ;
- R₁₈ est choisi parmi :
   - le radical 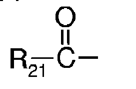
   - l'atome d'hydrogène ;

R₁₇, R₁₉ et R₂₁, identiques ou différents, sont choisis parmi les radicaux hydrocarbonés en C₁₃-C₁₇, linéaires ou ramifiés, saturés ou insaturés et de préférence parmi les radicaux alkyles et alcényle en C₁₃-C₁₇, linéaires ou ramifiés, saturés ou insaturés.
Avantageusement, les radicaux hydrocarbonés sont linéaires.

On peut citer par exemple comme composés de formule (X) les sels (chlorure ou méthylsulfate notamment) de diacyloxyéthyl diméthyl ammonium, de diacyloxyéthyl hydroxyéthyl méthyl ammonium, de monoacyloxyéthyl dihydroxyéthyl méthyl ammonium, de triacyloxyéthyl méthyl ammonium, de monoacyloxyéthyl hydroxyéthyl diméthyl ammonium et leurs mélanges. Les radicaux acyles ont de préférence 14 à 18 atomes de carbone et proviennent plus particulièrement d'une huile végétale comme l'huile de palme ou de tournesol. Lorsque le composé contient plusieurs radicaux acyles, ces derniers peuvent être identiques ou différents. Ces produits sont obtenus par exemple par estérification directe de la triéthanolamine, de la triisopropanolamine, d'alkyldiéthanolamine ou d'alkyldiiso-propanolamine éventuellement oxyalkylénées sur des acides gras ou sur des mélanges d'acides gras d'origine végétale ou animale ou par transestérification de leurs esters méthyliques. Cette estérification est suivie d'une quaternisation à l'aide d'un agent alkylant tel qu'un halogénure d'alkyle (méthyle ou éthyle de préférence), un sulfate de dialkyle (méthyle ou éthyle de préférence), le méthanesulfonate de méthyle, le paratoluènesulfonate de méthyle, la chlorhydrine du glycol ou du glycérol.

De tels composés sont par exemple commercialisés sous les dénominations **DEHYQUART** par la société **HENKEL, STEPANQUAT** par la société **STEPAN, NOXAMIUM** par la société **CECA, REWOQUAT WE** 18 par la société **REWO-WITCO.**

Quand elle contient des sels d'ammonium, la composition selon l'invention contient de préférence un mélange de sels de mono, di et triester d'ammonium quaternaire avec une majorité en poids de sels de diester.

Comme mélange de sels d'ammonium, on peut utiliser par exemple le mélange contenant 15 à 30 % en poids de méthylsulfate d'acyloxyéthyl dihydroxyéthyl méthyl ammonium, 45 à 60% de méthylsulfate de diacyloxyéthyl hydroxyéthyl méthyl ammonium et 15 à 30% de méthylsulfate de triacyloxyéthyl méthyl ammonium, les radicaux acyles ayant de 14 à 18 atomes de carbone et provenant d'huile de palme éventuellement partiellement hydrogénée.

On peut aussi utiliser les sels d'ammonium contenant au moins une fonction ester décrits dans les brevets US-A-4874554 et US-A-4137180.

Lorsque la nanoémulsion contient un ou plusieurs lipides amphiphiles ioniques, ils sont présents dans les nanoémulsions de l'invention, de préférence, dans des concentrations allant de 0,01 à 6 % en poids par rapport au poids total de la nanoémulsion et plus particulièrement de 0,2 à 4 % en poids.

Selon son caractère plus hydrophile ou plus lipophile, le lipide amphiphile non ionique ou ionique peut être introduit dans la phase aqueuse ou dans la phase huileuse de la nanoémulsion. La teneur totale de lipides amphiphiles non ioniques et ioniques peut, de préférence, aller de 0,25 à 15 % en poids et de préférence de 1 à 10 % en poids par rapport au poids total de la nanoémulsion.

La phase huileuse de la nanoémulsion selon l'invention comprend au moins une huile. Les huiles pouvant être utilisées dans les nanoémulsions de l'invention sont choisies préférentiellement dans le groupe formé par :
- les huiles d'origine animale ou végétale, formées par des esters d'acides gras et de polyols, en particulier les triglycérides liquides, par exemple les huiles de tournesol, de maïs, de soja, d'avocat, de jojoba, de courge, de pépins de raisin, de sésame, de noisette, les huiles de poisson, le tricaprocaprylate de glycérol, ou les huiles végétales ou animales de formule R₉COOR₁₀ dans laquelle R₉ représente le reste d'un acide gras supérieur comportant de 7 à 29 atomes de carbone et R₁₀ représente une chaîne hydrocarbonée linéaire ou ramifiée contenant de 3 à 30 atomes de carbone, en particulier alkyle ou alkényle, par exemple, l'huile de Purcellin ou la cire liquide de jojoba ;
- des huiles essentielles naturelles ou synthétiques telles que, par exemple, les huiles d'eucalyptus, de lavandin, de lavande, de vétivier, de litsea cubeba, de citron, de santal, de romarin, de camomille, de sarriette, de noix de muscade, de cannelle, d'hysope, de carvi, d'orange, de géraniol, de cade et de bergamote;
- les huiles de synthèse telles que l'huile de parléam, les polyoléfines et les esters d'acides carboxyliques liquides ;
- les huiles minérales telles que l'hexadécane, l'isohexadécane et l'huile de paraffine ;
- les huiles halogénés, notamment des fluorocarbures tels que des fluoramines par exemple la perfluorotributylamine, des hydrocarbures fluorés, par exemple le perfluorodécahydronaphtalène, des fluoroesters et des fluoroéthers ;
- les huiles de silicone volatiles ou non volatiles.

Les polyoléfines utilisables comme huiles de synthèse sont en particulier les poly-α-oléfines et plus particulièrement celles de type polybutène, hydrogéné ou non, et de préférence polyisobutène, hydrogéné ou non.

Les esters d'acides carboxyliques liquides utilisables comme huiles de synthèse, peuvent être des esters d'acides mono-, di-, tri- ou tétra-carboxyliques. Le nombre total de carbone des esters est généralement supérieur ou égal à 10 et de préférence inférieur à 100 et plus particulièrement inférieur à 80. Ce sont notamment les monoesters d'acides aliphatiques saturés ou insaturés, linéaires ou ramifiés en C₁-C₂₆ et d'alcools aliphatiques saturés ou insaturés, linéaires ou ramifiés en C₁-C₂₆ , le nombre total de carbone des esters étant généralement supérieur ou égal à 10. On peut également utiliser les esters d'acides di- ou tri-carboxyliques en C₄-C₂₂ et d'alcools en C₁-C₂₂ et les esters d'acides mono-, di- ou tri-carboxyliques et d'alcools di-, tri-, tétra- ou penta-hydroxylés en C₂-C₂₆.

Parmi les esters cités ci-dessus, on préfère utiliser les palmitates d'alkyle tels que le palmitate d'éthyle, le palmitate d'isopropyle, le palmitate d'éthyl-2-héxyle, le palmitate de 2-octyldécyle ; les myristates d'alkyle tels que le myristate d'isopropyle, le myristate de butyle, le myristate de cétyle, le myristate de 2-octyldodécyle ; les stéarates d'alkyle tel que le stéarate d'hexyle, le stéarate de butyle, le stéarate d'isobutyle ; les malates d'alkyle tels que le malate de dioctyle ; les laurates d'alkyle tels que le laurate d'hexyle et le laurate de 2-hexyldécyle ; l'isononanate d'isononyle ; l'octanoate de cétyle.

Avantageusement, la nanoémulsion selon l'invention contient au moins une huile de poids moléculaire supérieur ou égal à 400, notamment allant de 400 à 10 000, mieux allant de 400 à 5000, ou encore allant de 400 à 5000. Les huiles de poids moléculaire supérieur ou égal à 400 peuvent être choisies parmi les huiles d'origine animale ou végétale, les huiles minérales, les huiles de synthèse et les huiles de silicone, et leurs mélanges. Comme huiles de ce type, on peut citer par exemple le palmitate d'isocétyle, le stéarate d'isocétyle, l'huile d'avocat, l'huile de jojoba.

Les nanoémulsions conformes à l'invention comportent une quantité de phase huileuse (huile et autres corps gras hormis le lipide amphiphile) allant de préférence, de 2 à 40 % en poids par rapport au poids total de la nanoémulsion et plus particulièrement de 4 à 30 % en poids et préférentiellement de 4 à 20 % en poids.

Selon un mode particulier de réalisation de l'invention, les nanoémulsions conformes à l'invention comportent une phase huileuse comprenant une proportion d'huile(s) ayant un poids moléculaire supérieur ou égal à 400 représentant de préférence au moins 40 % en poids de la phase huileuse.

La phase huileuse et les lipides amphiphiles (amphiphiles non ioniques et ioniques) sont de préférence présents dans la nanoémulsion selon l'invention selon un rapport pondéral de la quantité de phase huileuse sur la quantité de lipide amphiphile allant de 3 à 10 et préférentiellement allant de 3 à 6. On entend ici par "quantité de phase huileuse" la quantité totale des constituants de cette phase huileuse sans inclure la quantité de lipide amphiphile.

Les nanoémulsions conformes à la présente invention peuvent contenir, en complément des dérvés d'urée de formule (I) décrits précédemment, des solvants, notamment pour améliorer, si nécessaire, la transparence de la composition.

Ces solvants sont choisis de préférence dans le groupe formé par :
- les alcools inférieurs en C₁-C₈ tels que l'éthanol ;
- les glycols tels que la glycérine, le propylèneglycol, le 1,3- butylèneglycol, le dipropylèneglycol, les polyéthylèneglycols comportant de 4 à 16 unités d'oxyde d'éthylène et de préférence de 8 à 12.
- les sucres tels que le glucose, le fructose, le maltose, le lactose, le sucrose.

Ces solvants peuvent être utilisés en mélange. Lorsqu'ils sont présents dans la nanoémulsion de l'invention, ils peuvent être utilisés à des concentrations allant de préférence de 0,01 à 30 % en poids par rapport au poids total de la nanoémulsion, et mieux de 5 à 20 % en poids par rapport au poids total de la nanoémulsion. La quantité d'alcool(s) et/ou de sucre(s) va de préférence de 5 à 20 % en poids par rapport au poids total de la nanoémulsion et la quantité de glycol(s) va de préférence de 5 à 15 % en poids par rapport au poids total de la nanoémulsion.

La nanoémulsion selon l'invention peut comprendre au moins un agent épaississant.

Les agents épaississants peuvent permettre d'augmenter la viscosité des nanoémulsions fluides (5 cP) d'au moins d'un facteur 5, pour une concentration en polymère égale à 1 % en poids.. Ajoutés à une nanoémulsion, ils permettent d'obtenir des compositions transparentes et stables, constituant des laits ou des crèmes. On entend par « lait » ou « crème » des compositions ayant une viscosité allant de 0,5 à 150 Poises (soit 0,05 Pa.s à 15 Pa.s) mesurée à 25°C avec Rheomat 180 au mobile 3, 4 ou 5 (selon la gamme de viscosité), à 200 s⁻¹.

L'agent épaississant peut être choisi parmi les esters ou les éthers de polyéthylène glycol ayant de 80 à 350 motifs d'oxyde d'éthylène, les polymères anioniques comportant au moins une chaîne hydrophobe, les polymères non ioniques hydrosolubles choisi parmi les homopolymères et copolymères d'oxyde d'éthylène ; les alcools polyvinyliques ; les homopolymères et copolymères de vinylpyrrolidone ; les homopolymères et copolymères de vinylcaprolactame ; les homopolymères et copolymères de polyvinylméthyléther ; les homopolymères et copolymères acryliques neutres ; les alkyl-C₁-C₂-celluloses et leurs dérivés ; les alkyl-C₁-C₃-guar ou hydroxyalkyl-C₁-C₃-guar.

L'agent épaississant peut être présent dans la nanoémulsion selon l'invention en une teneur allant de 0,005 % à 20 % en poids, par rapport au poids total de la composition, de préférence allant de 0,01 à 10 % en poids, et préférentiellement allant de 0,1 à 5 % en poids.

En particulier, les épaississants esters ou éthers de polyéthylène glycol peuvent être choisis parmi ceux répondant à la formule (XI) suivante :

R1-(O-CH2-CH2)n - OR2 (XI)

dans laquelle :
R1 désigne un groupement alkyle ou un groupement acyle ayant de 8 à 30 atomes de carbone, linéaire ou ramifié, saturé ou insaturé,
R2 désigne un atome d'hydrogène, un groupement alkyle ou un groupement acyle ayant de 1 à 30 atomes de carbone, linéaire ou ramifié, saturé ou insaturé,
n est un nombre compris entre 80 et 350.

De préférence R1 est un groupement acyle ayant de 12 à 20 atomes de carbone.
De préférence R2 est un groupement acyle ayant de 12 à 20 atomes de carbone.
De préférence n est un nombre compris entre 100 et 300.

De préférence, le rapport en poids de la partie hydrophile (-(O-CH2-CH2)nO) sur la partie hydrophobe (R1 et/ou R2) est compris entre 8 et 1000.

On utilise de préférence un composé de formule (XI) dans laquelle R1 et R2 désignent un goupement acyle ayant de 12 à 20 atome de carbone et n est compris entre 100 et 300. On peut par exemple citer le distéarate de PEG 150 et le distéarate de PEG 250.

De tels composés sont notamment vendus sous la dénomination Emanon 3299R par la société KAO et sous la dénomination KESSCO PEG 6000 DS par la société AKZO.

Les épaississants polymères anioniques comportant au moins une chaîne hydrophobe utilisés comme agent épaississant sont de préférence hydrosolubles ou hydrodispersibles, c'est-à-dire qu'ils sont solubles dans l'eau à un pH supérieur à 3,5. Ils comportent au moins une chaîne hydrophobe, ils sont non réticulés et ils ont de préférence un poids moléculaire allant de 10.000 à 2.000.000.

La ou les chaînes hydrophobes du polymère anionique utilisé sont notamment des chaînes hydrocarbonées linéaires ou ramifiées, saturées ou insaturées, ayant de 6 à 30 atomes de carbone, telles que alkyle, arylalkyle, alkylaryle, alcoylène ; des groupements divalents cycloaliphatiques tels que notamment méthylènedicyclohexyl et isophorone ; ou des groupements divalents aromatiques tels que phénylène.

Les polymères anioniques épaississants peuvent être choisis notamment parmi les copolymères d'acide acrylique ou méthacrylique, les copolymères d'acide 2-acrylamido-2-méthylpropane sulfonique et leurs mélanges. Les copolymères d'acide acrylique ou méthacrylique sont préférés. On entend par « copolymères » aussi bien les copolymères obtenus à partir de deux sortes de monomères que ceux obtenus à partir de plus de deux sortes de monomères tels que les terpolymères obtenus à partir de trois sortes de monomères.

Les polymères anioniques utilisés de préférence sont obtenus par copolymérisation d'un monomère (a) choisi parmi les acides carboxyliques à insaturation α,β-éthylénique (monomère a') et l'acide 2-acrylamido 2-méthylpropane sulfonique (monomère a"), avec un monomère (b) à insaturation éthylénique non tensioactif différent de (a) et/ou un monomère (c) à insaturation éthylénique issu de la réaction d'un monomère acrylique à insaturation α,β-monoéthylénique ou d'un monomère isocyanate à insaturation monoéthylénique avec un composant amphiphile non ionique monohydrique ou avec une amine grasse primaire ou secondaire.

Ainsi, les polymères anioniques utilisés peuvent être obtenus par deux voies de synthèses :
- soit par copolymérisation des monomères (a') et (c), ou (a'), (b) et (c), ou (a") et (c), ou (a"), (b) et (c),
- soit par modification (et notamment estérification ou amidification) d'un copolymère formé à partir des monomères (a') ou à partir des monomères (a') et (b), ou (a") et (b), par un composé amphiphile non ionique monohydrique ou une amine grasse primaire ou secondaire.

Comme copolymères d'acide 2-acrylamido 2-méthylpropane sulfonique, on peut citer notamment ceux décrits dans l'article « Miscelle formation of random copolymers of sodium 2-(acrylamido)-2-methylpropanesulfonate and a non-ionic surfactant macromonomer in water as studied by fluorescence and dynamic light scattering - Macromolecules 2000, Vol. 33, N° 10 - 3694-3704 » et dans les demandes EP-A-0 750 899 et EP-A-1,069,172.

L'acide carboxylique à insaturation α,β-monoéthylénique constituant le monomère (a') peut être choisi parmi de nombreux acides et en particulier parmi l'acide acrylique, l'acide méthacrylique, l'acide crotonique, l'acide itaconique et l'acide maléique. Il s'agit de préférence de l'acide acrylique ou méthacrylique.

Le copolymère peut contenir un monomère (b) à insaturation monoéthylénique qui n'a pas de propriété tensioactive. Les monomères préférés sont ceux qui donnent des polymères insolubles dans l'eau lorsqu'ils sont homopolymérisés. Ils peuvent être choisis par exemple parmi les acrylates et méthacrylates d'alkyle en C₁-C₄ comme l'acrylate de méthyle, l'acrylate d'éthyle, l'acrylate de butyle ou les méthacrylates correspondants. Les monomères plus particulièrement préférés sont l'acrylate de méthyle et l'acrylate d'éthyle. Les autres monomères pouvant être utilisés sont par exemple le styrène, le vinyltoluène, l'acétate de vinyle, l'acrylonitrile et le chlorure de vinylidène. Les monomères non réactifs sont préférés, ces monomères étant ceux dans lesquels le groupe éthylénique unique est le seul groupe réactif dans les conditions de la polymérisation. Toutefois, des monomères qui contiennent des groupes réactifs sous l'action de la chaleur comme l'acrylate d'hydroxyéthyle peuvent éventuellement être utilisés.

Le monomère (c) est obtenu par réaction d'un monomère acrylique à insaturation α,β-monoéthylénique tel que (a), ou d'un monomère isocyanate à insaturation monoéthylénique, avec un composé amphiphile non ionique monohydrique ou une amine grasse primaire ou secondaire.

Les composés amphiphiles non ioniques monohydriques ou les amines grasses, primaires ou secondaires, utilisés pour obtenir le monomère non-ionique (c) sont bien connus. Les composés amphiphiles non ioniques monohydriques sont généralement des composés hydrophobes alcoxylés contenant un oxyde d'alkylène formant la partie hydrophile de la molécule. Les composés hydrophobes sont généralement constitués par un alcool aliphatique ou un alkylphénol dans lesquels une chaîne carbonée contenant au moins six atomes de carbone constitue la partie hydrophobe du composé amphiphile.

Les composés amphiphiles non ioniques monohydriques préférés sont des composés ayant la formule (XII) suivante :

R-(OCH₂CHR')ₘ-(OCH₂CH₂)ₙ-OH (XII)

dans laquelle R est choisi parmi les groupes alkyle ou alcoylène comportant de 6 à 30 atomes de carbone et les groupes alkylaryle ayant des radicaux alkyle comportant de 8 à 30 atomes de carbone, R' est choisi parmi les groupes alkyle comportant de 1 à 4 atomes de carbone, n est un nombre moyen allant d'environ 1 à 150 et m est un nombre moyen allant d'environ 0 à 50, à la condition que n soit au moins aussi grand que m.

De préférence, dans les composés de formule (XII), le groupe R est choisi parmi les groupes alkyle comportant de 12 à 26 atomes de carbone et les groupes alkyl-(C₈-C₁₃)-phényle ; le groupe R' est le groupe méthyle ; m = 0 et n = 1 à 25.

Les amines grasses primaires et secondaires préférées sont constituées d'une ou deux chaînes alkyles comportant de 6 à 30 atomes de carbone.

Le monomère utilisé pour former le monomère uréthane non-ionique (c) peut être choisi parmi des composés très variés. On peut utiliser tout composé contenant une insaturation copolymérisable telle qu'une insaturation acrylique, méthacrylique ou allylique. Le monomère (c) peut être obtenu notamment à partir d'un isocyanate à insaturation monoéthylénique tel que notamment l'α,α-diméthyl-m-isopropényl-benzyl-isocyanate.

Le monomère (c) peut être en particulier choisi parmi les acrylates, méthacrylates ou itaconates d'alcool gras en C₆-C₃₀ oxyéthyléné (1 à 50 OE), tels que le steareth-20 méthacrylate, le méthacrylate de béhényle oxyéthyléné (25 OE), l'itaconate de mono-cétyle oxyéthyléné (20 OE), l'itaconate de mono-stéaryle oxyéthyléné (20 OE), l'acrylate modifié par des alcools en C12-C24 polyoxyéthylénés (25 OE), et parmi les diméthyl m-isopropénylbenzylisocyanates d'alcool gras en C₆-C₃₀ oxyéthyléné (1 à 50 OE), tel que notamment le diméthyl m-isopropénylbenzylisocyanate d'alcool béhénylique oxyéthyléné.

Selon un mode particulier de réalisation de l'invention, le polymère anionique est choisi parmi les terpolymères acryliques obtenus à partir de (a) un acide carboxylique à insaturation α,β-éthylénique, (b) un monomère à insaturation éthylénique non tensioactif différent de (a), et (c) un monomère uréthanne non-ionique qui est le produit de réaction d'un composé amphiphile non-ionique monohydrique avec un isocyanate à insaturation monoéthylénique.

Comme polymères anioniques comportant au moins une chaîne hydrophobe, pouvant être utilisés dans la nanoémulsion de l'invention, on peut citer notamment le terpolymère acide acrylique/acrylate d'éthyle/acrylate d'alkyle, tel que le produit en dispersion aqueuse à 30 % commercialisé sous la dénomination Acusol 823 par la société Rohm & Haas ; le copolymère acrylates/steareth-20 methacrylate tel que le produit commercialisé sous la dénomination Aculyn 22 par la société Rohm & Haas ; le terpolymère acide (méth)acrylique / acrylate d'éthyle / méthacrylate de béhényle oxyéthyléné (25 OE), tel que le produit en émulsion aqueuse commercialisé sous la dénomination Aculyn 28 par la société Rohm & Haas ; le copolymère acide acrylique/itaconate de mono-cétyle oxyéthyléné (20 OE), tel que le produit en dispersion aqueuse à 30 % commercialisé sous la dénomination Structure 3001 par la société National Starch ; le copolymère acide acrylique/itaconate de mono-stéaryle oxyéthyléné (20 OE) tel que le produit en dispersion aqueuse à 30 % commercialisé sous la dénomination Structure 2001 par la société National Starch ; le copolymère acrylates/acrylate modifié par des alcools en C12-C24 polyoxyéthylénés (25 OE), tel que le latex à 30-32 % de copolymère, commercialisé sous la dénomination Synthalen W2000 par la société 3V SA ; le terpolymère acide méthacrylique/acrylate de méthyle/diméthylmétaisopropényl benzylisocyanate d'alcool béhénylique éthoxylé, tel que le produit en dispersion aqueuse à 24 % et comportant 40 groupes oxyéthylénés, décrit dans le document EP-A-0 173 109.

L'addition de neutralisants peut s'avérer utile pour augmenter la solubilité des polymères dans l'eau. On peut alors utiliser tout neutralisant connu, et en particulier on peut le choisir parmi les bases inorganiques telles que la soude, la potasse, l'ammoniac, et parmi les bases organiques telles que la mono-, di- et triéthanolamine, l'aminométhylpropanediol-1,3, la N-méthylglucamine, les acides aminés basiques comme l'arginine et la lysine, et leurs mélanges. Le pH des compositions selon l'invention doit être supérieur à 4, et va de préférence de 5 à 8 et mieux de 5 à 7. La quantité de neutralisant dépend du polymère utilisé et des autres constituants de la formule. Elle peut aller par exemple de 0,01 à 5 % et mieux de 0,05 à 5 % du poids total de la composition.

Comme agent épaississant, on peut également utiliser un polymère non ionique hydrosoluble choisi parmi les homopolymères et copolymères d'oxyde d'éthylène ; les alcools polyvinyliques ; les homopolymères et copolymères de vinylpyrrolidone ; les homopolymères et copolymères de vinylcaprolactame ; les homopolymères et copolymères de polyvinylméthyléther ; les homopolymères et copolymères acryliques neutres ; les alkyl-C₁-C₂-celluloses et leurs dérivés ; les alkyl-C₁-C₃₋guar ou hydroxyalkyl-C₁-C₃-guar.

Les polymères utilisés selon la présente invention sont hydrosolubles, c'est-à-dire solubles dans l'eau, et non ioniques c'est-à-dire neutres.

Les polymères neutres hydrosolubles utilisés selon l'invention sont en particulier choisis parmi les polymères décrits ci-dessous et leurs mélanges.
A) les homopolymères et copolymères d'oxyde d'éthylène, ayant une masse molaire égale ou supérieure à 10 000 g/mole et de préférence allant 10 000 g/mole à 10 000 000 g/mole. Il peuvent être choisis parmi :
   (1) les poly-oxydes d'éthylène ayant la formule (XIII) suivante :

      R-(CH₂-CH₂-O)ₙ-R' (XIII)

      dans laquelle R est choisi parmi les groupes hydroxyle (OH), méthoxy (OCH₃) et amine (NH₂), R' est un groupe méthyl (CH₃) ou un hydrogène, et n est un nombre allant de 220 à 230 000.
   (2) les copolymères d'oxyde d'éthylène et d'un ou plusieurs monomères oxyalkylénés ayant la formule (XIV) suivante :

      -(CHR-CHR'-O)- (XIV)
   dans laquelle R et R' indépendamment l'un de l'autre sont un hydrogène ou un groupe alkyle comportant de 1 à 7 atomes de carbone, l'un au moins de R ou R' étant un groupe alkyle.
   Parmi les homopolymères et copolymères d'oxyde d'éthylène, on peut citer notamment les produits commercialisés sous les dénominations Polyox Coagulant (masse molaire d'environ 5.10⁶ g/mole) (nom CTFA : PEG-115M) et Polyox WSR N-60K CG (nom CTFA : PEG-45M) (masse molaire d'environ 2.10⁶ g/mole) par la société Amerchol, ainsi que le produit commercialisé sous la dénomination Carbowax 20M (nom CTFA : PEG-350) (masse molaire d'environ 2.10⁷ g/mole) par la société Union Carbide.
B) les alcools polyvinyliques, notamment ceux ayant une masse molaire moyenne allant de 10 000 g/mole à 500 000 g/mole. Ce sont des composés représentés par la formule (XV) suivante : 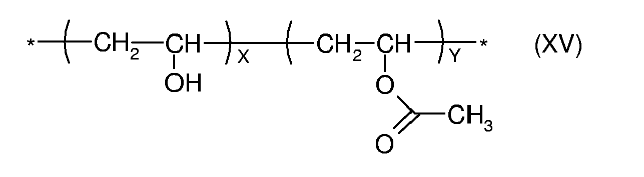 dans laquelle x est un nombre moyen exprimé en pourcentage allant de 70 à 100 ; y est un nombre moyen égal à 100 - x.
   On peut citer par exemple les produits commercialisés sous les dénominations Airvols 103, 350, 203, 540, 714 et 603 par la société Air Products.
C) les homopolymères et copolymères de vinylpyrrolidone, notamment ceux ayant une masse molaire moyenne allant de 10 000 g/mole à 1 000 000 g/mole. Ils peuvent être choisis parmi :
   1) les polyvinylpyrrolidones ayant la formule (XVI) suivante : 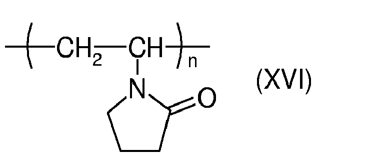 On peut citer par exemple les produits commercialisés sous les dénominations Polyclar V15 (masse molaire d'environ 8 000 g/mole), V30 (masse molaire d'environ 50 000 g/mole), V60 (masse molaire d'environ 400 000 g/mole), V90 (masse molaire d'environ 1 000 000 g/mole) et V120 (masse molaire d'environ 2 500 000 g/mole) par la société ISP.
   2) les copolymères de vinylpyrrolidone tels que :
      (a) les copolymères de vinylpyrrolidone et d'acétate de vinyle, notamment le copolymère contenant 30% d'acétate de vinyle, commercialisé sous la dénomination PVP-VA 735 par la société ISP ;
      (b) les copolymères de vinylpyrrolidone et de dérivés de vinylpyrrolidone à greffons butène comme le copolymère contenant 10 % de vinylpyrrolidone à greffons butène, commercialisé sous la dénomination Ganex (ou Antaron) P904 par la société ISP ;
      (c) les copolymères de vinylpyrrolidone et d'anhydride maléique (nom CTFA : PVM/MA copolymer), tels que les produits commercialisés sous les dénominations Gantrez AN-119 (masse molaire d'environ 190 000 g/mole), AN-139 (masse molaire d'environ 950 000 g/mole), AN-149 (masse molaire d'environ 1 100 000 g/mole), AN-169 (masse molaire d'environ 1 700 000 g/mole) et AN-179 (masse molaire d'environ 2 000 000 g/mole) par la société ISP ;
      (d) les copolymères de la vinylpyrrolidone avec les polyvinylalkyléthers de formule (XVII) suivante : 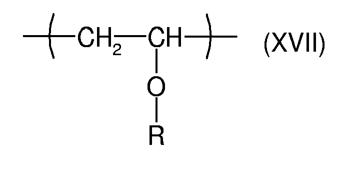 dans laquelle R est choisi parmi les groupes alkyle contenant de 1 à 7 atomes de carbone. De préférence, R est un groupe méthyle ;
      (e) les copolymères de vinylpyrrolidone et de N-vinyl lactames tel que le N-butyrolactame et le N-vinylcaprolactame ;
      (f) les copolymères de la vinylpyrrolidone avec les dérivés acryliques neutres de formule (XVIII) suivante : 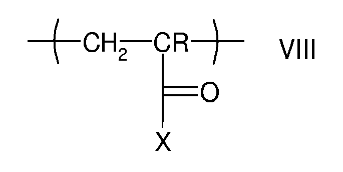 dans laquelle R est un hydrogène ou un groupe méthyl, et X est choisi parmi les groupes oxyde d'alkyle de type OR' où R' contient de 1 à 7 atomes de carbone ; oxyde d'alkyle hydroxylé et/ou aminé de type OR₁(OH)ₙ(NR₂R₃)ₘ où n et m sont des nombres allant de 0 à 10, R₁ est un groupe alkyle contenant de 1 à 7 atomes de carbone; R₂ et R₃ sont indépendamment l'hydrogène ou un groupe alkyle tel que la somme des atomes de carbone de R₂ et R₃ aille de 1 à 7 ; amine primaire, secondaire ou tertiaire de type NR₂R₃ où R₂ et R₃ ont la signification indiquée ci-dessus.
D) les homopolymères et copolymères de vinylcaprolactame qui peuvent être choisis parmi :
   1) les polyvinylcaprolactames qui ont la formule (XIX) suivante : 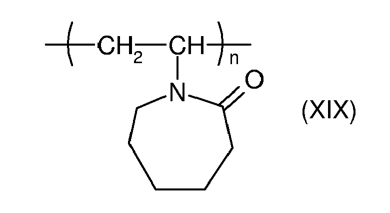
   2) les copolymères de vinylcaprolactames obtenus à partir de vinylcaprolactame et d'un ou plusieurs des monomères suivants :
      - acétate de vinyle ;
      - N-vinyl lactame tel que tel que le N-butyrolactame, le N-vinylcaprolactame et la N-vinylpyrrolidone ;
      - anhydride maléique ;
      - vinylalkyléthers de formule (XVII) indiquée ci-dessus;
      - dérivés acryliques neutres de formule (XVIII) indiquée ci-dessus.
   Comme polymères et copolymères de ce type, on peut citer par exemple le produit commercialisé sous la dénomination Luviskol Plus par la société BASF et le produit commercialisé sous la dénomination H2OLD EP-1 par la société ISP.
E) les homopolymères et copolymères de polyvinylméthyléther qui peuvent être choisis parmi :
   1) les polyvinylméthyléthers de formule (XVII) indiquée ci-dessus ;
   2) les copolymères obtenus à partir de vinylméthyléther et d'un ou plusieurs des monomères suivants :
      - vinylalkyléthers de formule (XVII) indiquée ci-dessus,
      - acétate de vinyle ;
      - N-vinyl lactame tel que le N-butyrolactame, le N-vinylcaprolactame et la N-vinylpyrrolidone ;
      - anhydride maléique ;
      - dérivés acryliques neutres de formule (XVIII) indiquée ci-dessus.
   Comme polymères et copolymères de ce type, on peut citer par exemple les produits commercialisés sous les dénominations Gantrez (nom CTFA : PVM/MA copolymer), et particulièrement Gantrez AN-119 (masse molaire d'environ 190 000 g/mole), AN-139 (masse molaire d'environ 950 000 g/mole), AN-149 (Masse molaire ≈ 1 100 000 g/mole), AN-169 (masse molaire d'environ 1 700 000 g/mole) et AN-179 (masse molaire d'environ 2 000 000 g/mole) par la société ISP.
F) les homopolymères et copolymères acryliques neutres, notamment ceux ayant une masse molaire allant de 10 000 g/mole à 5 000 000 g/mole. Ils peuvent être choisis parmi :
   1) les polymères acryliques hydrosolubles neutres ayant la formule (XX) suivante : 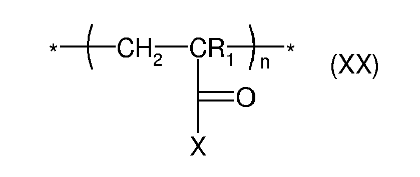 dans laquelle R₁ est un hydrogène ou un groupe méthyl, et X est choisi parmi (a) les groupes alkylaminés ou (b) les groupes oxydes d'alkyle hydroxylé et/ou aminé.
      Les polymères avec des groupes (a) alkylaminés sont des composés de formule (XX) où X = NR₂R₃ tels que le polymère acrylique correspondant soit hydrosoluble, R₂ et R₃ étant indépendamment un hydrogène ou un groupe alkyle tel que la somme des atomes de carbone de R₂ et R₃ aille de 1 à 7. Comme polymères de ce type, on peut citer notamment les polyacrylamides où R₁, R₂ et R₃ sont un hydrogène ; les polyméthylacrylamides où R₁ est un groupe méthyl et R₂ et R₃ sont un hydrogène ; les poly N-méthylacrylamides où R₁ et R₂ sont un hydrogène et R₃ est un groupe méthyl ; les poly N,N'-diméthylacrylamides où R₁ est un hydrogène et R₂ et R₃ sont un groupe méthyle ; les poly-N-éthylacrylamides où R₁ et R₂ sont un hydrogène et R₃ est un groupe éthyl ; les poly-N-isopropylacrylamides où R₁ et R₂ sont un hydrogène et R₃ est un groupe isopropyle.
      Comme polymère de ce type, on peut citer le polyacrylamide commercialisé sous la dénomination Superfloc N300 LMW par la société Cytec.
      Les polymères avec des groupes (b) oxydes d'alkyle hydroxylé et/ou aminé sont des composés de formule (XX) dans laquelle X = OR₂(OH)ₙ(NR₃R₄)ₘ où n et m sont des nombres allant de 0 à 10, R₂ est un groupe alkyle contenant de 1 à 7 atomes de carbone; R₃ et R₄ sont indépendamment l'hydrogène ou un groupe alkyle tel que la somme des atomes de carbone de R₃ et R₄ aille de 1 à 7, ces groupes étant tels que le dérivé acrylique correspondant soit hydrosoluble.
      Comme polymère de ce type, on peut citer le polyméthacrylate de glycéryle, commercialisé sous la dénomination Lubrajel CG par la société Guardian.
   2) les copolymères d'un dérivé acrylique hydrosoluble et neutre de formule (XX) tel précédemment décrit et d'un ou plusieurs monomère(s) neutre(s) suivants :
      - acétate de vinyle ;
      - N-vinyl lactame tel que le N-butyrolactame, le N-vinylcaprolactame et la N-vinylpyrrolidone ;
      - anhydride maléique ;
      - vinylalkyléthers de formule (XVII) indiquée ci-dessus ;
      - dérivé acrylique neutre de formule (XVIII) indiquée ci-dessus.
G) les alkyl-C₁-C₂-celluloses et leurs dérivés neutres, notamment ceux ayant une masse molaire allant de 10 000 g/mole à 5 000 000 g/mole. Ils peuvent être notamment choisis parmi l'hydroxyéthylcellulose comme le produit commercialisé sous les dénominations Natrosols 250 LR et 250 HHR par la société Aqualon ; l'éthylhydroxyéthylcellulose comme les produits commercialisés sous les dénominations Elfacos CD 481 et CD 411 par la société Akzo Nobel ; la méthylcellulose et les methylhydroxyalkylcelluloses comme le produit commercialisé sous la dénomination Methocel A4C par la société Dow Chemical et les produits commercialisés sous les dénominations Benecel par la société Hercules.
H) les alkyl-C₁-C₃-guar ou hydroxyalkyl-C₁-C₃-guar, notamment ceux ayant une masse molaire allant de 10 000 g/mole à 5 000 000 g/mole. On peut citer en l'hydroxypropylguar comme le produit commercialisé sous la dénomination Jaguar HP-105 par la société Rhodia.

Le procédé de préparation d'une nanoémulsion telle que définie ci-dessus consiste à mélanger la phase aqueuse contenant le dérivé d'urée et la phase huileuse, sous agitation vive, à une température allant de 10 °C à 80 °C, à effectuer une étape d'homogénéisation haute pression à une pression supérieure à 5.10⁷ Pa et à ajouter éventuellement le polymère utilisé. Selon un mode préféré de réalisation de l'invention, on effectue ensuite encore une étape d'homogénéisation haute pression à une pression supérieure à 5.10⁷ Pa. L'homogénéisation haute pression est réalisée de préférence à une pression allant de 6.10⁷ à 18.10⁷ Pa. Le cisaillement va de préférence de 2.10⁶ s⁻¹ à 5.10⁸ s⁻¹ et mieux de 1.10⁸ s⁻¹ à 3.10⁸ s⁻¹ (s⁻¹ signifie seconde⁻¹). Un tel procédé permet de réaliser des nanoémulsions compatibles avec des composés actifs thermosensibles, et pouvant contenir des huiles et notamment des parfums qui renferment des corps gras, sans les dénaturer.

Les nanoémulsions définies ci-dessus peuvent être utilisées dans tout domaine où ce type de composition est utile. Elles peuvent constituer notamment des compositions à usage topique et en particulier des compositions cosmétiques ou dermatologiques selon le type d'actifs et la quantité de ces actifs qu'elles comportent. Elles peuvent aussi être utilisées comme supports ophtalmiques.

Elles peuvent en outre constituer dans le domaine pharmaceutique le support d'une composition pharmaceutique qui peut être administrée par voie orale, parentérale ou transcutanée.

Une telle composition à usage topique, pharmaceutique ou ophtalmique contient un milieu physiologiquement acceptable, c'est-à-dire compatible avec la peau, les muqueuses, le cuir chevelu, les yeux et/ou les cheveux.

L'invention a aussi pour objet un support ophtalmique, caractérisé en ce qu'il contient une nanoémulsion telle que définie précédemment.

L'invention a aussi pour objet une composition pharmaceutique, caractérisée en ce qu'elle contient une nanoémulsion telle que définie précédemment.

Un autre objet de l'invention consiste en une composition cosmétique ou dermatologique, caractérisée en ce que qu'elle est constituée d'une nanoémulsion ou comprend une nanoémulsion telle que définie précédemment.

Les compositions de l'invention peuvent contenir des adjuvants et notamment des actifs hydrosolubles ou liposolubles, ayant une activité cosmétique ou dermatologique. Les actifs liposolubles sont dans les globules huileux de l'émulsion, tandis que les actifs hydrosolubles sont dans la phase aqueuse de l'émulsion. On peut citer, à titre d'exemples d'actif, les vitamines et leurs dérivés telles que la vitamine E et ses esters tels que l'acétate de vitamine E, la vitamine C et ses esters, les vitamines B, la vitamine A alcool ou rétinol et ses esters tels que le palmitate de vitamine A, la vitamine A acide ou acide rétinoïque et ses dérivés, les provitamines telles que le panthénol, la niacinamide, l'ergocalciférol, les anti-oxydants, les huiles essentielles, les humectants, les filtres solaires, les agents hydratants, les protéines, les céramides et les pseudocéramides, la DHEA et ses dérivés et précurseurs biologiques. Comme adjuvants, on peut citer aussi les séquestrants, les conservateurs, les charges, les filtres UV, les adoucissants, les matières colorantes (pigments ou colorants) et les parfums.

Comme actifs ophtalmiques, on peut citer par exemple les agents anti-glaucome, tels que le betaxolol ; les antibiotiques tels que l'acyclovir ; les antiallergiques ; les agents anti-inflammatoires tels que l'ibuprofène et ses sels, le diclofénac et ses sels, l'indométhacine ; les agents antiviraux.

Les quantités de ces différents adjuvants sont celles classiquement utilisées dans le domaine considéré, et par exemple de 0,01 à 20 % du poids total de la composition. Ces adjuvants ainsi que leurs concentrations doivent être tels qu'ils ne modifient pas la propriété recherchée pour la composition de l'invention.

L'invention a donc également pour objet une composition cosmétique, dermatologique, pharmaceutique ou ophtalmologique comprenant une nanoémulsion selon l'invention, et en particulier une composition cosmétique.

La nanoémulsion de l'invention peut être par exemple utilisée pour le soin, le traitement, le maquillage des matières kératiniques (notamment d'être humain), en particulier de la peau (notamment du visage et/ou du cuir chevelu) et des lèvres.

L'invention a donc aussi pour objet l'utilisation cosmétique de la nanoémulsion telle que définie ci-dessus pour le soin, le traitement et/ou le maquillage de la peau, notamment du visage et/ou du cuir chevelu, et des lèvres.

La composition selon l'invention peut être un produit pour le soin de la peau, notamment pour le visage, le cou, le contour de l'oeil, le corps.

La composition de maquillage comprenant la nanoémulsion selon l'invention peut être un produit de maquillage des lèvres (rouge à lèvres), un fond de teint, un fard à joues, un fard à paupières, un eye-liner, un produit anti-cernes, un produit de maquillage du corps.

Avantageusement, la composition est une composition non rincée.

En outre, la nanoémulsion de l'invention peut aussi être utilisée pour le soin et/ou le traitement des cheveux. Elle permet d'obtenir un dépôt d'huile sur les cheveux, ce qui rend ceux-ci plus brillants, plus résistants au coiffage, sans toutefois les alourdir. Elle permet aussi, en prétraitement d'améliorer les effets de la coloration ou de la permanente.

L'invention a donc aussi pour objet l'utilisation cosmétique de la nanoémulsion telle que définie ci-dessus pour le soin et/ou le traitement des cheveux.

La nanoémulsion selon l'invention permet notamment une bonne hydratation de la peau, des muqueuses et/ou du cuir chevelu, et est particulièrement adaptée au traitement de la peau sèche.

Un autre objet de l'invention est donc un procédé cosmétique de soin et/ou d'hydratation et/ou de maquillage de la peau, des muqueuses et/ou du cuir chevelu, caractérisé en ce qu'on applique sur la peau, les muqueuses et/ou le cuir chevelu une nanoémulsion telle que définie ci-dessus.

L'invention porte également sur l'utilisation de la nanoémulsion selon l'invention pour la fabrication d'une composition destinée au traitement de la peau sèche.

Enfin, l'invention porte aussi sur l'utilisation de la nanoémulsion selon l'invention pour la fabrication d'une composition ophtalmologique.

Les exemples qui suivent, permettront de mieux comprendre l'invention, sans toutefois présenter un caractère limitatif. Les quantités indiquées sont en % en poids sauf mention contraire.

Les compositions selon l'invention peuvent se présenter sous forme d'une émulsion, notamment d'émulsions huile-dans-eau (H/E) ou eau-dans-huile (E/H) ou multiple (triple : E/H/E ou H/E/H). Ces compositions sont préparées selon les méthodes usuelles. De préférence, la composition est une émulsion huile-dans-eau.

En outre, les compositions utilisées selon l'invention peuvent être plus ou moins fluides et avoir l'aspect d'une crème blanche ou colorée, d'une pommade, d'un lait, d'une lotion, d'un sérum.

L'invention est illustrée plus en détails par les exemples non limitatifs décrits ci-après.

Le mode opératoire suivant est mis en oeuvre :
- dans une première phase A, on homogénéise les lipides amphiphiles avec les huiles et les actifs et adjuvants lipophiles à une température d'environ 45°C ;
- dans une seconde phase B, on dissout les actifs et adjuvants hydrophiles à une température de 20 à 30°C ;
- puis, on mélange les phases A et B à l'aide d'un homogénéisateur à turbine puis on homogénéise à l'aide d'un homogénéisateur à haute pression du type Soavi-Niro à une pression de 1200 bars, en 7 passages en maintenant la température du produit en dessous de 45 °C.

### Exemple 1 :

On prépare un soin hydratant ayant la composition suivante :

### Première phase :

- Isostéarate de PEG-400, vendu par la société UNICHEMA 4,5 %
- Sel disodique de l'acide N-stéaroyl L-glutamique commercialisé sous la dénomination Acylglutamate HS21 par la Société AJINOMOTO (lipide amphiphile ionique) 0,5 %
- Huile de jojoba 5 %
- Huile d'avocat 5 %
- Cyclométhicone 9%
- Ethanol absolu non dénaturé 7,5 %

### Deuxième phase :

- N-(2-hydroxyéthyl)-urée 7,5 %
- Eau déminéralisée 61 %

On obtient une émulsion stable dont la taille des globules d'huile est inférieure à 100 nm.

Le composition appliquée sur la peau ne présente pas de sensation de collant et procure un bon effet hydratant.

### Exemple 2 :

On prépare une eau parfumée ayant la composition suivante :

### Première phase :

- Mélange de palmitate/stéarate de sucrose vendu sous la dénomination Crodesta F70 par la société CRODA 4,5 %
- Sel disodique de l'acide N-stéaroyl L-glutamique commercialisé sous la dénomination Acylglutamate HS21 par la Société AJINOMOTO (lipide amphiphile ionique) 0,5 %
- Huile de soja 6%
- Huile de silicone volatile 2%
- Parfum 3 %
- Acétate de vitamine E 0 ,5 %
- Ethanol absolu non dénaturé 10%

### Deuxième phase :

- N-(2-hydroxyéthyl)-urée 5 %
- Eau déminéralisée 68,5 %

On obtient une émulsion stable dont la taille des globules d'huile est inférieure à 100 nm.

Le composition appliquée sur la peau ne présente pas de sensation de collant et procure un bon effet hydratant.

### Exemple 3 :

On prépare un lait démaquillant non rincé ayant la composition suivante :

### Première phase :

- Tristéarate de sorbitane oxyéthyléné (20 OE) vendu sous la dénomination Tween 65 par la société UNICHEMA 4,5 %
- Sel disodique de l'acide N-stéaroyl L-glutamique commercialisé sous la dénomination Acylglutamate HS21 par la Société AJINOMOTO (lipide amphiphile ionique) 0,5 %
- Stéarate d'isocétyle 5 %
- Myristate d'isopropyle 10 %
- Cyclométhicone 5%
- Ethanol absolu non dénaturé 7,5 %

### Deuxième phase :

- N-(2-hydroxyéthyl)-urée 7,5 %
- Eau déminéralisée 61 %

On obtient une émulsion stable dont la taille des globules d'huile est inférieure à 100 nm.

Le composition s'applique facilement sur la peau, ne présente pas de sensation de collant et procure un bon effet hydratant.

## Revendications

1. Nanoémulsion huile-dans-eau comportant une phase huileuse dispersée dans une phase aqueuse, dont les globules d'huile ont une taille moyenne en nombre inférieure à 100 nm, **caractérisée en ce qu'**elle comprend :
- (i) au moins un lipide amphiphile comprenant au moins un lipide amphiphile non ionique et éventuellement un lipide amphiphile ionique, la phase huileuse et le lipide amhiphile étant présents en une teneur telle que le rapport pondéral phase huileuse/lipide amphiphile va de 3 à 10,
- et (ii) au moins un composé de formule (I) suivante : 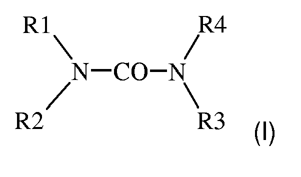
dans laquelle :
R₁, R₂, R₃ et R₄ représentent chacun, indépendamment l'un de l'autre, un atome d'hydrogène, un groupe alkyl en C₁-C₄ ou un groupe hydroxyalkyl en C₂-C₆ pouvant contenir de 1 à 5 groupes hydroxyles, où au moins un des radicaux R₁ à R₄ représente un groupe hydroxyalkyle,
ainsi que leurs sels, leurs solvats, et leurs isomères.

2. Nanoémulsion selon la revendication précédente, **caractérisée par le fait que** pour les composés de formule (I) R₁ désigne un groupe hydroxyalkyle en C₂-C₆ et R₂, R₃, R₄ désignent, indépendamment l'un de l'autre, un atome d'hydrogène ou un groupe alkyle en C₁-C₄.

3. Nanoémulsion selon l'une quelconque des revendications précédentes, **caractérisée en ce que** pour les composés de formule (I) R₁ désigne un groupe hydroxyalkyle en C₂-C₆ comprenant de 1 à 5 groupes hydroxyles, et R₂, R₃, R₄ désignent un atome d'hydrogène.

4. Nanoémulsion selon la revendication précédente, **caractérisée en ce que** R₁ désigne un groupe hydroxyalkyle en C₂-C₆ comprenant 1 groupe hydroxyle.

5. Nanoémulsion selon l'une quelconque des revendications précédentes, **caractérisée par le fait que** pour les composés de formule (I) R₁ désigne un groupe hydroxyalkyle en C2-C4 comprenant 1 groupe hydroxyle et R₂, R₃, R₄ désignent un atome d'hydrogène.

6. Nanoémulsion selon l'une quelconque des revendications précédentes, **caractérisée par le fait que** le composé de formule (I) est choisi parmi le N-(2-hydroxyéthyl)-urée ; le N-(2-hydroxypropyl)-urée ; le N-(3-hydroxypropyl)-urée ; le N-(2,3-dihydroxypropyl)- urée ; le N-(2,3,4,5,6-pentahydroxyhexyl)- urée ; le N-méthyl-N-(1,3,4,5,6-pentahydroxy-2-hexyl)- urée ; le N-méthyl-N'-(1-hydroxy-2-méthyl-2-propyl)- urée ; le N-(1-hydroxy-2-méthyl-2-propyl)- urée ; le N-(1,3-dihydroxy-2-propyl)- urée ; le N-(tris-hydroxyméthyl-méthyl)-urée ; le N-éthyl-N'-(2-hydroxyéthyl)- urée ; le N,N-bis-(2-hydroxyéthyl)- urée ; le N,N'-bis-(2-hydroxyéthyl)- urée ; le N,N-bis-(2-hydroxypropyl)- urée ; le N,N'-Bis-(2-hydroxypropyl)- urée ; le N,N-Bis-(2-hydroxyéthyl)-N'-propyl- urée ; le N,N-Bis-(2-hydroxypropyl)-N'-(2-hydroxyéthyl)- urée ; le N-tert.Butyl-N'-(2-(hydroxyéthyl)-N'-(2-(hydroxypropyl)- urée ; le N-(1,3-dihydroxy-2-propyl)-N'-(2-hydroxyéthyl)- urée ; le N,N-Bis-(2-hydroxyéthyl)-N',N'-dimethyl- urée ; le N,N,N',N'-tetrakis-(2-hydroxyéthyl)- urée ; le N',N'-Bis-(2-hydroxyéthyl)-N',N'-bis-(2-hydroxypropyl)-urée ; et leurs mélanges.

7. Nanoémulsion selon l'une quelconque des revendications précédentes, **caractérisée par le fait que** le composé de formule (I) est le N-(2-hydroxyéthyl)-urée.

8. Nanoémulsion selon l'une quelconque des revendications précédentes, **caractérisée par le fait que** les sels des composés de formule (I) sont choisis parmi les sels de l'acide sulfurique, l'acide chlorhydrique, l'acide bromhydrique, l'acide iodhydrique, l'acide phosphorique, l'acide borique, l'acide propionique, l'acide acétique, l'acide téréphtalique, l'acide citrique, l'acide tartrique.

9. Nanoémulsion selon l'une quelconque des revendications précédentes, **caractérisée par le fait que** le composé de formule (I) est présent en une teneur allant de 1 % à 50 % en poids, par rapport au poids total de la composition, de préférence allant de 2 % à 25 % en poids, et préférentiellement allant de 2 % à 20 % en poids.

10. Nanoémulsion selon la revendication précédente, **caractérisée par le fait que** le rapport pondéral phase huileuse / lipide amphiphile va de 2 à 6.

11. Nanoémulsion selon l'une quelconque des revendications précédentes, **caractérisée par le fait que** les globules d'huile ont une taille moyenne en nombre allant de 20 à 80 nm.

12. Nanoémulsion selon l'une quelconque des revendications précédentes, **caractérisée en ce que** le lipide amphiphile non ionique est choisi parmi :
1/ les tensioactifs siliconés,
2/ les lipides amphiphiles liquides à température inférieure ou égale à 45°C choisis parmi les esters d'au moins un polyol et d'au moins un acide gras comportant au moins une chaîne alkyle en C₈-C₂₂, saturée ou non saturée, linéaire ou ramifiée,
3/ les esters d'acide gras et de sucre et les éthers d'alcool gras et de sucre,
4/ les tensioactifs solides à une température égale à 45°C, choisis parmi les esters gras de glycérol, les esters gras de sorbitan et les esters gras de sorbitan oxyéthylénés, les éthers gras éthoxylés et les esters gras éthoxylés,
5/ les copolymères blocs d'oxyde d'éthylène et d'oxyde de propylène,
et leurs mélanges.

13. Nanoémulsion selon l'une quelconque des revendications précédentes, **caractérisée en ce que** le lipide amphiphile non ionique est présent en une teneur allant de 0,2 % à 12 % en poids par rapport au poids total de la composition, et de préférence allant de 0,2 % à 8 % en poids.

14. Nanoémulsion selon l'une quelconque des revendications précédentes, **caractérisée en ce qu'**elle comprend au moins un lipide amphiphile ionique.

15. Nanoémulsion selon la revendication précédente, **caractérisée par le fait que** le lipide amphiphile ionique additionnel est choisi parmi :
- les sels alcalins du dicétyl- et du dimyristylphosphate ;
- les sels alcalins du cholestérol sulfate ;
- les sels alcalins du cholestérol phosphate ;
- les lipoaminoacides et leurs sels ;
- les sels de sodium de l'acide phosphatidique ;
- les phospholipides ;
- les dérivés alkylsulfoniques ;
- les sels d'ammonium quaternaire
- les amines grasses et leurs sels.

16. Nanoémulsion selon la revendication 16 ou 17, **caractérisée en ce que** le lipide amphiphile ionique est présent en une teneur allant de 0,01 % à 6 % en poids par rapport au poids total de la composition, et de préférence allant de 0,2 % à 4 % en poids.

17. Nanoémulsion selon l'une quelconque des revendications précédentes, **caractérisée par le fait que** la teneur totale de lipides amphiphiles non ioniques et ioniques va de 0,25 % à 15 % en poids, par rapport au poids total de la composition, et de préférence va de 1 % à 10 % en poids.

18. Nanoémulsion selon l'une quelconque des revendications précédentes, **caractérisée en ce que** la quantité de phase huileuse va de 2 % à 40 % en poids par rapport au poids total de la composition.

19. Nanoémulsion selon l'une quelconque des revendications précédentes, **caractérisée en ce qu'**elle contient au moins une huile de poids moléculaire supérieur ou égal à 400, notamment allant de 400 à 10 000.

20. Nanoémulsion selon la revendication précédente, **caractérisée en ce que** la phase huileuse comprend une proportion d'huile(s) ayant un poids moléculaire supérieur ou égal à 400 représentant au moins 40 % en poids de la phase huileuse.

21. Nanoémulsion selon l'une quelconque des revendications précédentes, **caractérisée par le fait qu'**elle comprend un agent épaississant.

22. Nanoémulsion selon la revendication précédente, **caractérisée par le fait que** l'agent épaississant est choisi parmi les esters ou les éthers de polyéthylène glycol ayant de 80 à 350 motifs d'oxyde d'éthylène, les polymères anioniques comportant au moins une chaîne hydrophobe, les polymères non ioniques hydrosolubles choisi parmi les homopolymères et copolymères d'oxyde d'éthylène ; les alcools polyvinyliques ; les homopolymères et copolymères de vinylpyrrolidone ; les homopolymères et copolymères de vinylcaprolactame ; les homopolymères et copolymères de polyvinylméthyléther ; les homopolymères et copolymères acryliques neutres ; les alkyl-C₁-C₂-celluloses et leurs dérivés ; les alkyl-C₁-C₃-guar ou hydroxyalkyl-C₁-C₃-guar.

23. Nanoémulsion selon la revendication 21 ou 22, **caractérisée par le fait que** l'agent épaississant est présent en une teneur allant de 0,005 % à 20 % en poids, par rapport au poids total de la composition, de préférence allant de 0,01 à 10 % en poids, et préférentiellement allant de 0,1 à 5 % en poids.

24. Nanoémulsion selon l'une quelconque des revendications précédentes, **caractérisée en ce qu'**elle a une turbidité allant de 60 à 400 NTU.

25. Composition cosmétique ou dermatologique, **caractérisée en ce qu'**elle est constituée d'une nanoémulsion ou comprend une nanoémulsion selon l'une quelconque des revendications précédentes.

26. Composition selon la revendication précédente, **caractérisée par le fait qu'**elle comprend au moins un additif choisi parmi les vitamines, les provitamines, les anti-oxydants, les huiles essentielles, les humectants, les filtres solaires, les agents hydratants, les protéines, les céramides et les pseudocéramides, la DHEA et ses dérivés et précurseurs biologiques, les séquestrants, les conservateurs, les charges, les filtres UV, les adoucissants, les matières colorantes , les parfums.

27. Composition cosmétique selon la revendication 25 ou 26, **caractérisée par le fait que** la composition est une composition de soin de la peau ou une composition de maquillage de la peau.

28. Support ophtalmique, **caractérisé en ce qu'**il contient une nanoémulsion selon l'une quelconque des revendications 1 à 24.

29. Composition pharmaceutique, **caractérisée en ce qu'**elle contient une nanoémulsion selon l'une quelconque des revendications 1 à 24.

30. Utilisation cosmétique de la nanoémulsion selon l'une quelconque des revendications 1 à 24 ou de la composition selon la revendication 26, pour le soin, le traitement et/ou le maquillage de la peau, du visage et/ou du cuir chevelu.

31. Utilisation cosmétique de la nanoémulsion selon l'une quelconque des revendications 1 à 24 ou de la composition selon la revendication 25 ou 26, pour le soin et/ou le traitement des cheveux.

32. Procédé cosmétique de soin et/ou d'hydratation de la peau, des muqueuses et/ou du cuir chevelu, **caractérisé en ce qu'**on applique sur la peau, les muqueuses et/ou le cuir chevelu, une nanoémulsion selon l'une quelconque des revendications 1 à 24 ou une composition selon la revendication 25 ou 26.

33. Procédé cosmétique de maquillage de la peau comprenant l'application sur la peau d'une composition selon la revendication 27.

34. Utilisation de la nanoémulsion selon l'une quelconque des revendications 1 à 24, pour la fabrication d'une composition destinée au traitement de la peau sèche.

35. Utilisation de la nanoémulsion selon l'une quelconque des revendications 1 à 26, pour la fabrication d'une composition ophtalmologique.
